# EUROPÄISCHE PATENTSCHRIFT

(11) **EP 0 947 213 B1**
(45) Veröffentlichungstag und Bekanntmachung des Hinweises auf die Patenterteilung: **04.12.2002**
(21) Anmeldenummer: 99102600.6
(22) Anmeldetag: 11.02.1999
(51) Int. Cl.: A61M 39/22, A61M 1/28

(54) **Patientenkonnektor**
Patient connector
Raccord de patient

(30) Priorität: 30.03.1998 DE 19814047
(43) Veröffentlichungstag der Anmeldung: 06.10.1999
(73) Patentinhaber: Fresenius Medical Care Deutschland GmbH, 61350 Bad Homburg v.d.H. (DE)
(72) Erfinder: Döpper, Joachim Dr., 64521 Gross-Gerau (DE); Schulz, Wolfgang, 66606 St. Wendel (DE)
(74) Vertreter: Vièl, Christof, Dipl.-Ing.

(56) Entgegenhaltungen:
- EP-A- 0 576 380
- CH-A- 670 955
- FR-A- 2 604 237
- US-A- 4 291 706
- US-A- 4 306 705
- US-A- 5 540 265
- US-A- 5 730 418

## Beschreibung

Die Erfindung betrifft einen Patientenkonnektor für die Peritonealdialyse, bestehend aus einem Grundkörper, der einen ersten Stutzen zum Anschließen eines Behälters für Dialyselösung und einen weiteren Stutzen zur Verbindung des Patientenkonnektors mit einem Katheterkonnektor aufweist, wobei Mittel zum Abdichten der Stutzen vorgesehen sind.

Ein derartiger Patientenkonnektor ist aus der DE 44 43714 C2 bekannt, in der ein Gehäuse zwischen den Schläuchen für die frische und die verbrauchte Dialyselösung und dem Katheterkonnektor angeordnet ist, wobei durch das Betätigen von in dem Gehäuse angeordneten Verschlußstücken die einzelnen Schritte zum Austausch der Dialyseflüssigkeit in dem Bauchraum des Patienten erfolgen. Auf das Verschlußstück wird hierbei über eine nur in einer Richtung drehbare Anordnung eine radiale Bewegung übertragen. Diese Vorrichtung ist vom Aufbau her relativ komplex.

Aus der EP 576 380 A1, die als nächstliegender Stand der Technik augesehen wird, ist ein für die Peritonealdialyse geeigneter Patientenkonnektor bekannt, der aus einem Grundkörper besteht, welcher einen ersten Stutzen zum Anschließen eines Behälters für Dialyselösung und einen weiteren Stutzen zur Verbindung des Patientenkonnektors mit einem Katheterkonnektor aufweist, wobei Mittel zum Abdichten der Stutzen vorgesehen sind und an dem Grundkörper ein Druckknopf vorgesehen ist, wobei durch Betätigen des Druckknopfes der zweite Stutzen durch ein linear verschließbares Verschlußstück verschließbar und der erste Stutzen abdichtbar ist.

Aufgabe der Erfindung ist es somit, einen Patientenkonnektor für die Peritonealdialyse zu schaffen, der einen einfacheren Aufbau aufweist.

Diese Aufgabe wird erfindungsgemäß dadurch gelöst, daß der erste Stutzen zwischen einem Betätigungsorgan und einem Verschlußstück seitlich in den im wesentlichen linear verlaufenden Grundkörper mündet, daß der weitere Stutzen, das Verschlußstück und ein Dichtelement axial hintereinander in dem Grundkörper angeordnet sind, wobei das Betätigigungsorgan nicht mit dem Verschlußstück verbunden ist, und daß durch das Betätigungsorgan eine lineare Bewegung des Dichtelementes zum Verschließen des ersten Stutzens und des Verschlußstücks zum Verschließen des zweiten Stutzens einleitbar ist, wobei die einmal eingeleitete lineare Verschiebung irreversibel ist.

Der Erfindung liegt die Erkenntnis zugrunde, daß es möglich ist, eine Peritonealdialyse während des Schlafes des Patienten durchzuführen, indem Ventile die Ein- und Ausläufe der Lösung in das und aus dem Peritoneum steuern. Da in diesem Falle die Ventile die Schläuche freigeben und verschließen, ist ein Drei-Wege-Ventil, wie es in der DE 44 43 714 C2 beschrieben wird, nicht mehr erforderlich, da nur noch eine Leitung zum Peritonealkatheter führt. Ist die Peritonealdialyse beendet, wird der Patient mit Hilfe des erfindungsgemäßen Patientenkonnektors einfach, sicher und schnell dekonnektiert.

Es kann vorteilhaft sein, daß das Betätigungsorgan mit dem Dichtelement verbunden ist.

Es ist auch möglich, daß das Betätigungsorgan mit dem Dichtelement nicht verbunden ist.

Erfindungsgemäß ist auch, daß das Verschlußstück vor Betätigen des Betätigungsorganes in einem Käfig gehaltert ist, so daß Flüssigkeit über den ersten Stutzen durch den Grundkörper in den zweiten Stutzen durchtreten kann.

Eine Weiterbildung der Erfindung besteht darin, daß das Dichtelement im wesentlichen zylindrisch ausgebildet ist, wobei es am vorderen und am hinteren Ende Dichtlippen aufweist.

Es ist vorteilhaft, daß die Länge des Dichtelementes größer ist als der Durchmesser der Mündung des ersten Stutzens.

Die Vorteile der Erfindung bestehen im wesentlichen darin, daß ein Patientenkonnektor geschaffen wird, der einen einfacheren Aufbau und eine leichtere Bedienbarkeit aufweist als bekannte Patientenkonnektoren und der somit eine optimale Lösung für die ventilgesteuerte Peritonealdialyse während des Schlafes des Patienten darstellt.

Im folgenden wird ein Ausführungsbeispiel der Erfindung anhand von Zeichnungen beschrieben.

Es zeigen
- Fig. 1: einen erfindungsgemäßen Patientenkonnektor vor seiner Betätigung,
- Fig. 2: einen erfindungsgemäßen Patientenkonnektor nach seiner Betätigung.

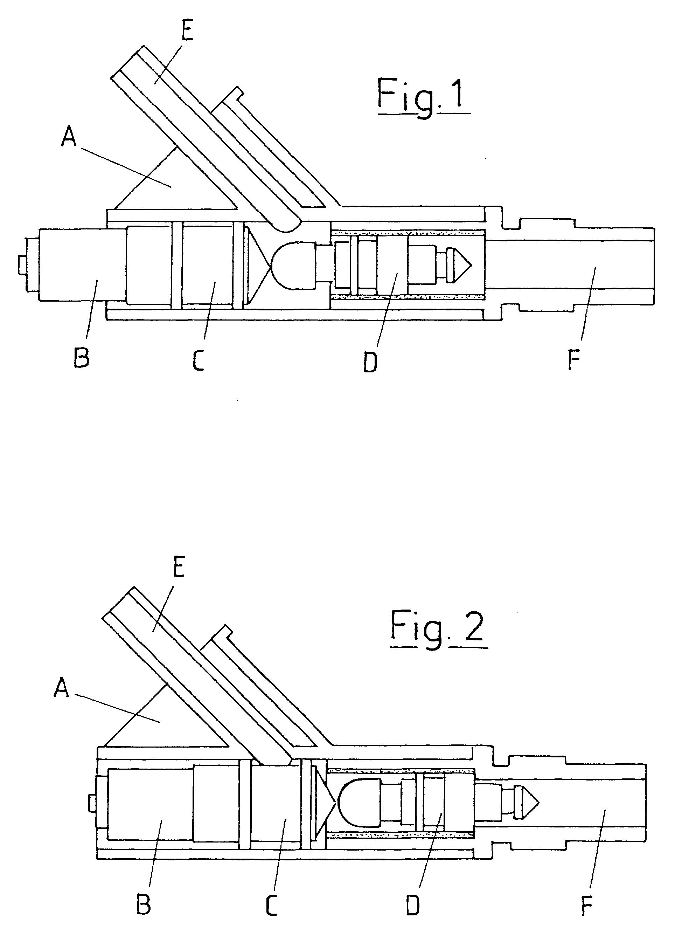

Der Patientenkonnektor gemäß Fig. 1 besteht aus einem im wesentlichen linearen Grundkörper A, der einen ersten Stutzen E für einen Behälter mit Dialyselösung und einen zweiten Stutzen F für die Verbindung des Patientenkonnektors mit einem Katheterkonnektor aufweist. In der Achse des zweiten Stutzens F, der in der Achse des Grundkörpers A angeordnet ist, ist ein Verschlußstück D in einem Käfig gehaltert, so daß Flüssigkeit über den ersten Stutzen E durch den Grundkörper A in den zweiten Stutzen F und somit von dem Behälter in den Katheterkonnektor durchtreten kann.

Hinter dem Verschlußstück D ist ein Dichtelement C angeordnet, das im wesentlichen zylindrisch ausgebildet ist und an dem vorderen und dem hinteren Ende Dichtlippen aufweist. Die Länge des Dichtelementes C ist größer als der Durchmesser der seitlichen Mündung des ersten Stutzens E in den Grundkörper A, so daß das Dichtelement diese Mündung verschließen kann. In dem in Fig. 1 dargestellten Zustand verschließt das Dichtelement den hinteren Bereich des im wesentlichen zylindrischen Grundkörpers A, in dem axial der zweite Stutzen F, das Verschlußstück D und das Dichtelement C hintereinander angeordnet sind.

Im vorliegenden Beispiel ist das Dichtelement C auf einen Druckknopf als Betätigungsorgan B aufgesteckt, das den hinteren Bereich des Grundkörpers A abschließt. Möglich ist auch, daß das Betätigungsorgan ein schraubbares Betätigungsorgan ist.

Bei Betätigung des Betätigungsorganes B (Fig. 2) erfolgt eine lineare Bewegung der in dem Grundkörper A angeordneten Elemente C und D. Hierbei wird das Verschlußstück D aus seinem Käfig in den zweiten Stutzen F geschoben und verschließt diesen. Gleichzeitig schiebt sich das Dichtelement C vor die Mündung des ersten Stutzens E und sperrt durch seine beiden Dichtlippen den ersten Stutzen E ab. Somit ist auch der hintere Bereich des Grundkörpers A abgedichtet.

Die Betätigung des Betätigungsorganes B ist irreversibel, d.h. das Betätigungsorgan B kann nach einmaliger Betätigung nicht nochmals betätigt werden und die einmal eingeleitete lineare Verschiebung der in dem Grundkörper A angeordneten Elemente ist endgültig.

## Patentansprüche

1. Patientenkonnektor für die Peritonealdialyse, bestehend aus einem Grundkörper (A), der einen ersten Stutzen (E) zum Anschließen eines Behälters für Dialyselösung und einen weiteren Stutzen (F) zur Verbindung des Patientenkonnektors mit einem Katheterkonnektor aufweist, wobei Mittel zum Abdichten der Stutzen vorgesehen sind, **dadurch gekennzeichnet, daß** der erste Stutzen (E) zwischen einem Betätigungsorgan (B) und einem Verschlußstück (D) seitlich in den im wesentlichen linear verlaufenden Grundkörper (A) mündet, daß der weitere Stutzen (F), das Verschlußstück (D) und ein Dichtelement (C) axial hintereinander in dem Grundkörper (A) angeordnet sind, wobei das Betätigigungsorgan (B) nicht mit dem Verschlußstück (D) verbunden ist, und daß durch das Betätigungsorgan (B) eine lineare Bewegung des Dichtelementes (C) zum Verschließen des ersten Stutzens (E) und des Verschlußstücks (D) zum Verschließen des weiteren Stutzens (F) einleitbar ist, wobei die einmal eingeleitete lineare Verschiebung irreversibel ist.

2. Patientenkonnektor gemäß Anspruch 1, **dadurch gekennzeichnet, daß** das Betätigungsorgan (B) mit dem Dichtelement (C) verbunden ist.

3. Patientenkonnektor gemäß Anspruch 1, **dadurch gekennzeichnet, daß** das Betätigungsorgan (B) mit dem Dichtelement (C) nicht verbunden ist.

4. Patientenkonnektor gemäß Anspruch 1, **dadurch gekennzeichnet, daß** das Verschlußstück (D) vor Betätigen des Betätigungsorganes (B) in einem Käfig gehaltert ist, so daß Flüssigkeit über den ersten Stutzen (E) durch den Grundkörper (A) in den zweiten Stutzen (F) durchtreten kann.

5. Patientenkonnektor gemäß Anspruch 1, **dadurch gekennzeichnet, daß** das Dichtelement (C) im wesentlichen zylindrisch ausgebildet ist, wobei es am vorderen und am hinteren Ende Dichtlippen aufweist.

6. Patientenkonnektor gemäß Anspruch 5, **dadurch gekennzeichnet, daß** die Länge des Dichtelementes (C) größer ist als der Durchmesser der Mündung des ersten Stutzens (E).

## Claims

1. A patient connector for peritoneal dialysis, consisting of a basic component (A) with a first connecting piece (E) for connection to a reservoir containing dialysis solution and another connecting piece (F) for connection of the patient connector to a catheter connector, means being provided for sealing off the connecting pieces,
**characterised in that** the first connecting piece (E) opens laterally into the essentially linear basic component (A) between an actuating member (B) and a closure element (D), that the other connecting piece (F), the closure element (D) and a sealing element (C) are arranged axially one behind the other in the basic component (A), the actuating member (B) not being connected with the closure element (D), and that by means of the actuating member (B), linear movement of the sealing element (C) can be initiated to close off the first connecting piece (E), and of the closure element (D) to close off the other connecting piece (F), the linear displacement being irreversible once it has been initiated.

2. The patient connector of claim 1, **characterised in that** the actuating member (B) is connected with the sealing element (C).

3. The patient connector of claim 1, **characterised in that** the actuating member (B) is not connected with the sealing element (C).

4. The patient connector of claim 1, **characterised in that** prior to actuation of the actuating member (B), the closure element (D) is contained in a cage, so that solution entering via the first connecting piece (E) can flow though the basic component (A) into the second connecting piece (F).

5. The patient connector of claim 1, **characterised in that** the sealing element (C) is essentially cylindrical and has sealing lips at the front and rear ends.

6. The patient connector of claim 5, **characterised in that** the sealing element (C) is longer than the diameter of the aperture through which the first connecting piece (E) opens into the main component (A).

## Revendications

1. Raccord de patient pour la dialyse péritonéale, composé d'un corps principal (A) muni d'une première tubulure (E) pour raccorder un récipient pour solution de dialyse et une tubulure supplémentaire (F) pour raccorder le raccord de patient avec un raccord de cathéter, des moyens étant prévus pour rendre étanche les tubulures, **caractérisé en ce que** la première tubulure (E) débouche latéralement dans le corps principal essentiellement linéaire entre un organe d'actionnement (B) et un organe d'obturation (D), **en ce que** la tubulure supplémentaire (F), l'organe d'obturation (D) et un élément d'étanchéité (C) sont placés les uns derrière les autres de façon axiale dans le corps principal (A), l'organe d'actionnement (B) n'étant pas relié à l'organe d'obturation (D), et **en ce qu'**un déplacement linéaire de l'élément d'étanchéité (C) pour obturer la première tubulure (E) et de l'organe d'obturation (D) pour obturer la tubulure supplémentaire (F) peut être initié par l'organe d'actionnement (B), le coulissement linéaire une fois initié étant irréversible.

2. Raccord de patient selon la revendication 1, **caractérisé en ce que** l'organe d'actionnement (B) est relié à l'élément d'étanchéité (C).

3. Raccord de patient selon la revendication 1, **caractérisé en ce que** l'organe d'actionnement (B) n'est pas relié à l'élément d'étanchéité (C).

4. Raccord de patient selon la revendication 1, **caractérisé en ce que** l'organe d'obturation (D), avant que l'organe d'actionnement ne soit actionné, est maintenu dans une cage de sorte que du liquide peut s'écouler de la première tubulure (E) dans la deuxième tubulure (F) en traversant le corps principal (A).

5. Raccord de patient selon la revendication 1, **caractérisé en ce que** l'organe d'étanchéité (C) a une forme essentiellement cylindrique et il est muni de lèvres d'étanchéité à ses extrémités avant et arrière.

6. Raccord de patient selon la revendication 5, **caractérisé en ce que** la longueur de l'élément d'étanchéité est plus grande que le diamètre de l'embouchure de la première tubulure (E).
